# EUROPEAN PATENT APPLICATION

(11) **EP 4 474 475 A1**
(43) Date of publication of application: **11.12.2024**
(21) Application number: 23747132.1
(22) Date of filing: 30.01.2023
(51) Int. Cl.: C12N 5/0783, C12M 1/00, C12M 1/12

(54) **PRODUCTION METHOD FOR NATURAL KILLER CELLS**

(30) Priority: 31.01.2022 JP 2022013646
(71) Applicant: Healios K.K., Tokyo 100-0006 (JP)
(72) Inventor: KURACHI, Kenji, Tokyo 100-0006 (JP); YAMADA, Masashi, Tokyo 100-0006 (JP); KIMURA, Hironobu, Tokyo 100-0006 (JP); TAMURA, Kouichi, Tokyo 100-0006 (JP)
(74) Representative: Weickmann & Weickmann PartmbB
(86) International application number: PCT/JP2023/002794
(87) International publication number: WO 2023/145922

(57) **Abstract**

The present invention provides a novel method for producing natural killer (NK) cells, which enables production of a large amount of high-quality NK cells easily, inexpensively, and safely in a short period of time from pluripotent stem cells. Specifically, a method for producing NK cells is provided, including
(1) a step of forming pluripotent stem cell spheres having an average particle size of not less than 200 um in a first medium;
(2) a step of inducing the pluripotent stem cell spheres formed in step (1) into a cell population including hematopoietic progenitor cells by three-dimensional culture using a second medium; and
(3) a step of inducing the cell population including hematopoietic progenitor cells obtained in step (2) into a cell population including NK cells by three-dimensional culture using a third medium,
wherein the steps (1) to (3) are performed by a perfusion culture method.

## Description

### [Technical Field]

The present invention relates to methods for producing natural killer cells (hereinafter sometimes to be abbreviated as "NK cells") useful in the medical field from pluripotent stem cells efficiently in large amounts.

### [Background Art]

There are many reports on methods for inducing NK cells from pluripotent stem cells. In the examples reported to date, the differentiation induction step into NK cells and the subsequent expansion culture step require a considerable amount of time, and plate culture (two-dimensional culture) using a culture dish and the like is the mainstream (e.g., Non Patent Literatures 1 and 2). Patent Literature 1 describes a method for inducing NK cells from pluripotent stem cell spheres via hematopoietic progenitor cells. However, the average particle size of the pluripotent stem cell spheres described in Patent Literature 1 is small, and medium exchange by perfusion culture is not described at all.

### [Citation List]

### [Patent Literature]

[Patent Literature 1]
WO2020/086889

### [Non Patent Literature]

[Non Patent Literature 1]
   Biochemical and Biophysical Research Communications Volume 515, Issue 1, 12 July 2019, Pages 1-8
[Non Patent Literature 2]
   Methods Mol Biol. 2019; 2048: 107-119.

### [Summary of Invention]

### [Technical Problem] .

When NK cells are induced according to the previously reported examples, the differentiation induction step and the expansion culture step require a considerable amount of time. Furthermore, since it is difficult to mechanize the operation of changing the culture medium, there is a risk that the quality of the final product may be affected by manual work. In addition, an increase in the production cost is also expected due to an increase in the number of steps required for changing the culture medium. In particular, in the case of two-dimensional culture, scaling up of the culture size has a theoretical limit. In this background, an NK cell induction technique that is more efficient than conventional techniques and permits scaling up is demanded.

### [Solution to Problem]

The present inventors have conducted various studies and found that NK cells can be induced efficiently and quickly by setting the average particle size of the pluripotent stem cell spheres formed in three-dimensional culture to not less than 200 µm and combining with a perfusion culture method in this state. In addition, it was found that the NK cells produced by this method have high activity and maintain the activity even after freezing and thawing, and therefore, they can be useful as an active ingredient of cell medicines.

Based on these findings, the present inventors have conducted further studies and completed the present invention.

Accordingly, the present invention provides the following.

### [Item 1]

A method for producing a natural killer (NK) cell, comprising
(1) a step of forming pluripotent stem cell spheres having an average particle size of not less than 200 um in a first medium;
(2) a step of inducing the pluripotent stem cell spheres formed in step (1) into a cell population including hematopoietic progenitor cells by three-dimensional culture using a second medium; and
(3) a step of inducing the cell population including hematopoietic progenitor cells obtained in step (2) into a cell population including NK cells by three-dimensional culture using a third medium,
wherein the steps (1) to (3) are performed by a perfusion culture method.

### [Item 2]

The method of Item 1, wherein the steps (1) to (3) are performed without using a three-dimensional culture carrier or an extracellular substrate.

### [Item 3]

The method of Item 2, wherein the perfusion culture in step (1) is performed using a separation membrane having a fine pore size of 15 - 75 um.

### [Item 4]

The method of Item 2, wherein the perfusion culture in step (2) is performed using a separation membrane having a fine pore size of 45 - 225 µm.

### [Item 5]

The method of Item 2, wherein the perfusion culture in step (3) is performed using a separation membrane having a fine pore size of 0.2 - 10 µm.

### [Item 6]

The method of any one of Items 1 to 5, wherein the steps (1) to (3) are performed by a continuous perfusion culture method.

### [Item 7]

The method of any one of Items 1 to 6, wherein the first medium comprises a ROCK inhibitor.

### [Item 8]

The method of any one of Items 1 to 7, wherein the second medium comprises VEGF, BMP4, and a GSK3β inhibitor.

### [Item 9]

The method of Item 8, wherein the second medium further comprises a ROCK inhibitor or bFGF.

### [Item 10]

The method of any one of Items 1 to 7, wherein the second medium comprises SCF, a TGFβ/Smad inhibitor, and VEGF.

### [Item 11]

The method of Item 10, wherein the second medium further comprises a ROCK inhibitor or bFGF.

### [Item 12]

The method of any one of Items 1 to 7, wherein the second medium comprises SCF and Flt3L.

### [Item 13]

The method of Item 12, wherein the second medium further comprises at least one selected from a ROCK inhibitor, IL-3, and IL-7.

### [Item 14]

The method of any one of Items 1 to 7, wherein the three-dimensional culture in step (2) comprises
(2-1) a culture step using a medium comprising VEGF, BMP4, and a GSK3β inhibitor as the second medium,
(2-2) a culture step using a medium comprising SCF, a TGFβ/Smad inhibitor, and VEGF as the second medium, and
(2-3) a culture step using a medium comprising SCF and Flt3L as the second medium.

### [Item 15]

The method of any one of Items 1 to 14, wherein the third medium comprises IL-15 and SCF.

### [Item 16]

The method of Item 15, wherein the third medium further comprises IL-7 and Flt3L.

### [Item 17]

The method of any one of Items 1 to 16, which does not require a step of sorting NK cells.

### [Advantageous Effects of Invention]

The present invention is characterized by the efficient production of large amounts of NK cells at a time by a production method using three-dimensional perfusion culture. The NK cells produced by the method of the present invention exhibit high cytotoxic activity against various cancer cells, and the activity is maintained without decrease even when frozen, thus enabling large-scale clinical application.

### [Brief Description of Drawings]

[Fig. 1]

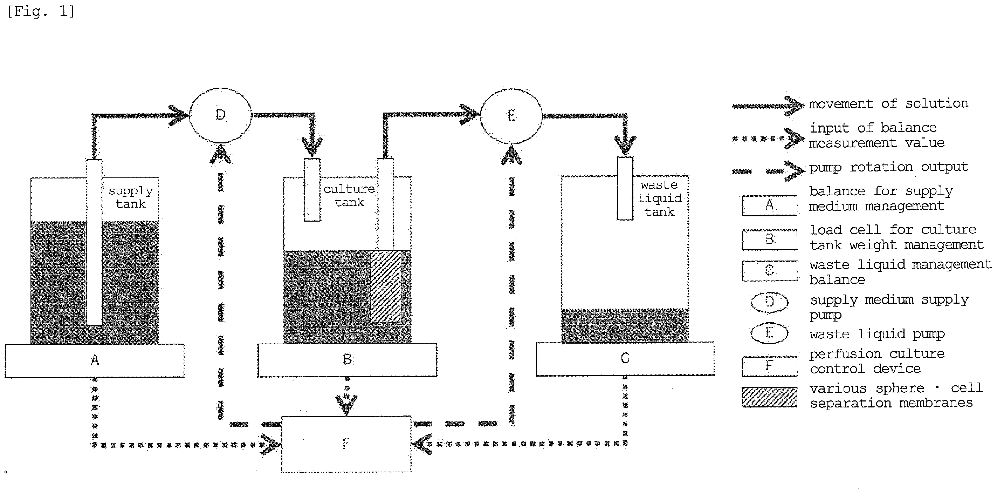

   Fig. 1 is a schematic diagram showing a typical example of a perfusion culture apparatus in the present invention.
[Fig. 2]

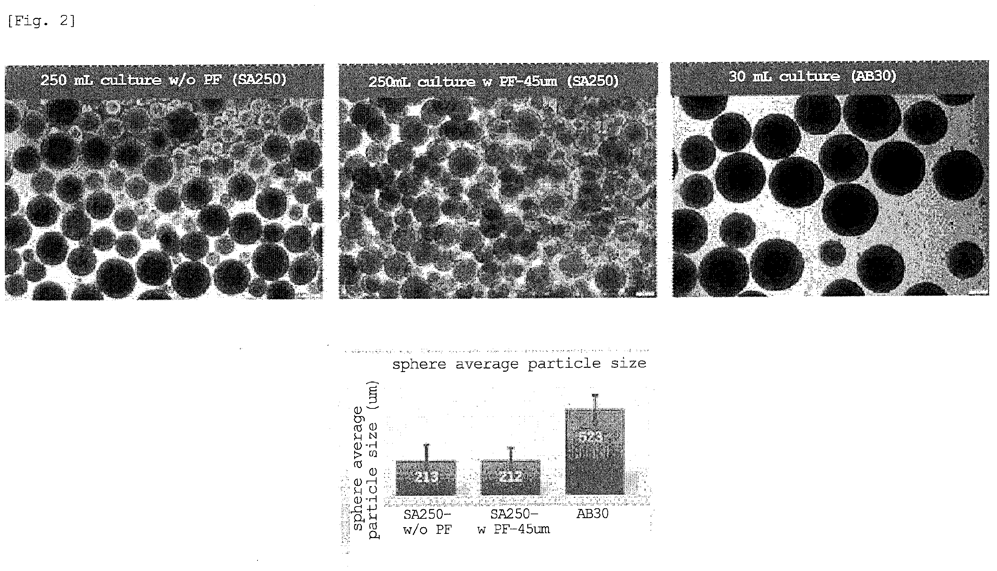

   Fig. 2 shows microscopic photographs of spheres on day 7 when human iPS cells were floating cultured under various conditions (from the left: 250 mL culture with manual medium exchange; perfusion culture, 250 mL culture; 30 mL culture with manual medium exchange) and a diagram showing the average particle size of the spheres obtained under each condition.
[Fig. 3]

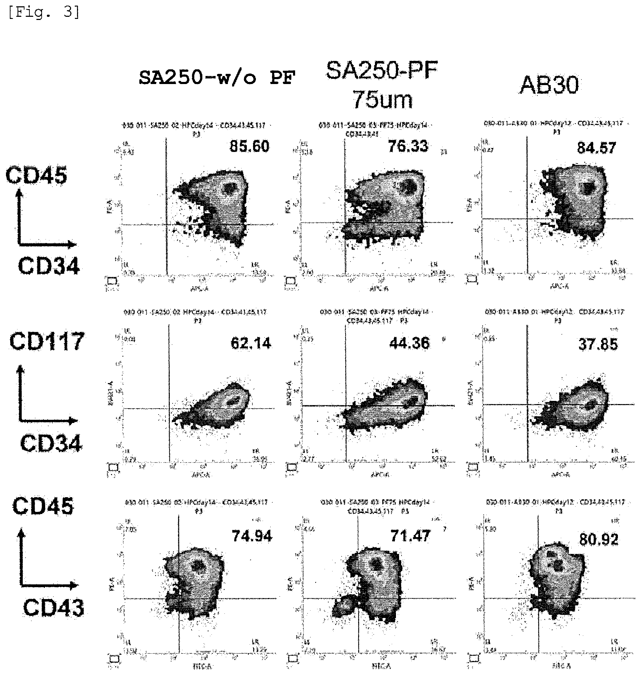

   Fig. 3 is a diagram showing the expression frequency of various hematopoietic progenitor cell marker surface molecules on day 14 of culture, when the iPS cell spheres obtained in step (1) were floating cultured under various conditions and induced to differentiate into hematopoietic progenitor cells (from the left: 250 mL culture with manual medium exchange; perfusion culture, 250 mL culture; 30 mL culture with manual medium exchange).
[Fig. 4]

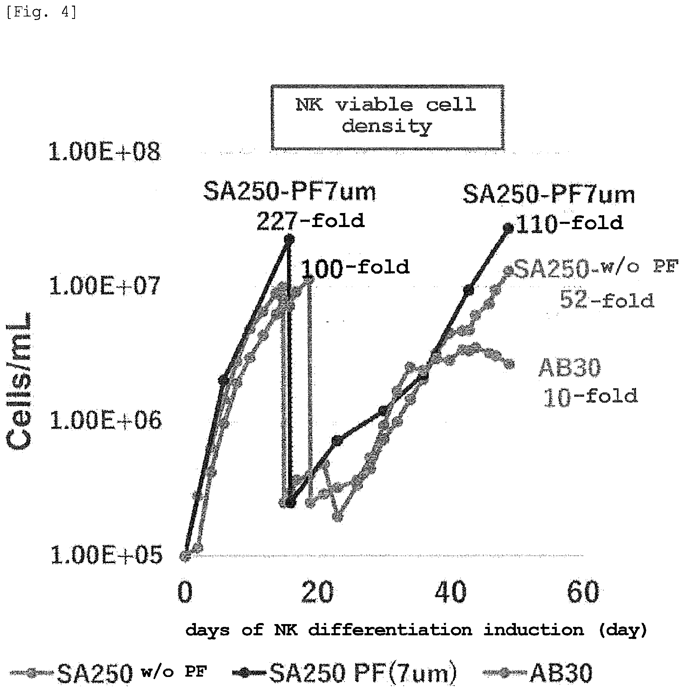

   Fig. 4 is a diagram showing daily changes in viable NK cell density when the hematopoietic progenitor cells obtained in step (2) were floating cultured under various conditions and induced to differentiate into NK cells. The decrease in cell density in the middle of the diagram was because the cells were reseeded into a new container.
[Fig. 5]

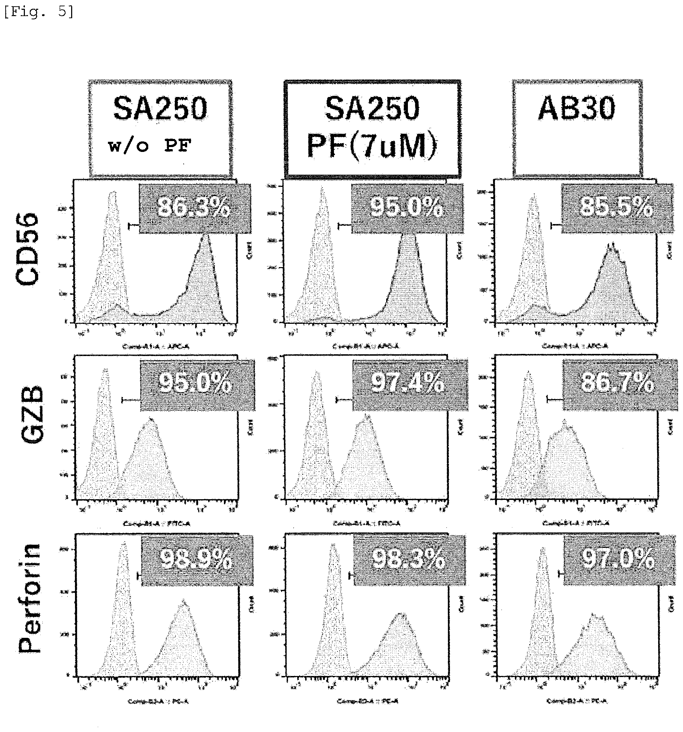

   Fig. 5 is a diagram showing the measurement results of the expression of the NK cell marker surface molecule (CD56) and granzyme B (GZB) and perforin involved in cytotoxic activity, after freezing and thawing the NK cell populations obtained by floating culture of the hematopoietic progenitor cells obtained in step (2) under various conditions (from the left: 250 mL culture with manual medium exchange; perfusion culture, 250 mL culture; 30 mL culture with manual medium exchange).
[Fig. 6]

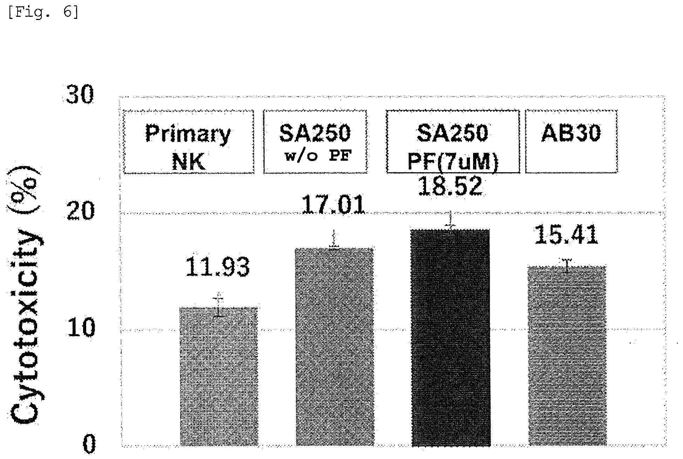

   Fig. 6 is a diagram showing the measurement results of the cytotoxic activity against A549 cells by LDH assay, after freezing and thawing the NK cells obtained in step (3) and further statically culturing the cells (from the left: primary NK cells isolated from human peripheral blood mononuclear cells (positive control); 250 mL culture with manual medium exchange; perfusion culture, 250 mL culture; 30 mL culture with manual medium exchange).

### [Description of Embodiments]

The present invention is a method for producing NK cells, including
(1) a step of forming pluripotent stem cell spheres having an average particle size of not less than 200 µm in a first medium;
(2) a step of inducing the pluripotent stem cell spheres formed in step (1) into a cell population including hematopoietic progenitor cells by three-dimensional culture using a second medium; and
(3) a step of inducing the cell population including hematopoietic progenitor cells obtained in step (2) into a cell population including NK cells by three-dimensional culture using a third medium,
wherein the steps (1) to (3) are performed by a continuous perfusion culture method (hereinafter sometimes to be referred to as "the method of the present invention").

### (Three-dimensional culture method)

The three-dimensional culture method in the present invention refers to a method in which cells are first formed into spheres (spheroids) and then cultured by making them suspended in a medium (sometimes to be referred.to as "the three-dimensional floating culture method" in the present specification).

The three-dimensional floating culture method is one of the cell culture methods and characterized by culturing floating cells, spheres (spheroids), or carriers for three-dimensional culture with cells attached thereto, while developing them three-dimensionally using a stirring blade or shaker. Therefore, the three-dimensional floating culture can maximize the number of cells per space as compared to two-dimensional culture, in which cells are only cultured on the bottom surface of the culture container. Furthermore, it can make the environment of the culture medium uniform by mixing three-dimensionally using a stirring blade or shaker. In the three-dimensional culture, efficient culture in a uniform environment is possible by combining with various detectors such as pH sensor, dissolved oxygen sensor, and the like.

The facts that the culture space can be utilized to the maximum extent and that culture can be performed in a uniform environment are also useful for scaling up of culture assuming commercial production. In addition, when culturing floating cells or spheroids, this culture method can be an advantageous culture method in terms of cost because it does not require specially processed plastic carriers for cell culture or an extracellular matrix required for many cultured cells. Furthermore, it is also one of the characteristics that culture can be performed in an environment closer to that of the living body than two-dimensional culture, in which cells are cultured by adhering them to a flat surface. Thus, it is a useful means for producing various cell products derived from pluripotent stem cells (e.g., embryonic stem (ES) cells, induced pluripotent stem (iPS) cells, and the like) that require differentiation induction into various cells.

### (Perfusion culture method)

The three-dimensional culture method of the present invention can more effectively induce NK cells when combined with a perfusion culture method by.

The perfusion culture method is one of the continuous culture methods, and is a method capable of achieving continuous supply of new nutrients and removal of waste products, which are the purposes of medium exchange, by simultaneously supplying a given amount of new medium to the culture medium in the culture container and removing a given amount of the medium.

The use of perfusion culture method eliminates the need for medium exchange, which is expected to drastically reduce the workload. In addition, because this method is generally performed mechanically, it does not cause environmental changes such as temperature change, pH change, dissolved oxygen concentration change, and dissolved carbon dioxide concentration change that occur with manual medium exchange. This enables a longer-term culture in the same container, and as a result, the cell density that can be maintained in the same container can be maintained at a high level. In addition, the perfusion culture method can also maintain a constant concentration of various growth factors, and the like secreted by cells, which are removed by general medium exchange, and is also a culture method that enables culture in an in vivo-like environment that mimics the blood circulation system.

The separation membrane used in perfusion culture desirably has a certain fine pore size and properties (e.g., hydrophobicity, etc.) that prevent the target cell population from escaping to the outside of the culture system. The fine pore size of the separation membrane can be appropriately selected according to the target cell population, but since various differentiated cells are induced inside and outside the sphere in the NK cell induction step, the selection of the fine pore size generally requires various considerations, and optimization thereof is very difficult.

The inventors have completed an efficient NK cell induction method according to the present invention by determining an appropriate fine pore size for each of the above-mentioned culture steps through numerous verifications. While specific fine pore sizes for each step are described later, an appropriate fine pore size for the perfusion culture of the present invention is a fine pore size of 15 - 75 um for step (1), a fine pore size of 45 - 225 um for step (2), and a fine pore size of 0.2 - 10 µm for step (3).

While the material of the separation membrane is not particularly limited, and a material that does not affect the cells or the components in the culture medium is preferably used. Examples of such material include metal materials such as SUS304, SUS316, and the like, natural fibers such as cellulose fiber and the like, and chemical fibers such as polysulfone fiber, polyethersulfone fiber, and the like.

The three-dimensional culture method of the present invention is characterized in that a cell population, such as cell spheres or cells, present in a culture system is perfusion cultured in each step described below. The conditions for each perfusion culture are as described below for each step. The perfusion culture in the present invention permits "continuous perfusion culture".

The "continuous perfusion culture" here refers to the process of simply replacing the membrane between each step, without a washing step generally performed when exchanging the medium, and supplying the medium for the next step while removing the medium from the previous step.

For example, in step (1), the medium is continuously exchanged by supplying the first medium described below while simultaneously withdrawing the medium, and when switching from step (1) to step (2), the medium is continuously exchanged from the first medium to the second medium by supplying the second medium described below while simultaneously withdrawing the first medium. In step (2), the medium is continuously exchanged by supplying the second medium while simultaneously withdrawing the medium, and when switching from step (2) to step (3), the medium is continuously exchanged from the second medium to the third medium by supplying the third medium described below while simultaneously withdrawing the second medium.

### (Step of forming pluripotent stem cell spheres: step (1))

Step (1) in the method of the present invention is a step of forming pluripotent stem cell spheres having an average particle size of not less than 200 um in the first medium.

The pluripotent stem cells used in the present invention for forming pluripotent stem cell spheres include, but are not limited to, ES cell and iPS cell. For example, when using iPS cells, the method of producing iPS cells and the cells from which they are derived are not particularly limited. The method of culturing iPS cells is also not particularly limited, and may be two-dimensional culture or three-dimensional culture. Furthermore, cryopreserved iPS cells may also be used.

The sphere formation is performed under floating culture conditions. The floating culture conditions are not particularly limited as long as the environment of the culture medium can be made uniform by three-dimensional mixing using a stirring blade or shaker. The container used for floating culture is not particularly limited, and a container with a stirring mechanism such as a stirring blade agitator or a stirring blade up-and-down agitator is used. The seeding density of the pluripotent stem cells in the sphere formation of the pluripotent stem cells of the present invention is within the range of 1.0×10⁴ cells/mL to 1.0×10⁶ cells/mL. When the density is higher than this, the shape of the sphere may become too large, which may affect the subsequent induction efficiency. Among them, the cells are more preferably seeded within the range of 5.0×10⁴ cells/mL to 2.0×10⁵ cells/mL.

The culture medium used for the formation of pluripotent stem cell spheres in this step (1) is not particularly limited as long as it is a culture medium used for the maintenance culture of pluripotent stem cells. Examples of such culture medium include media capable of feeder-free culture, such as StemFit (registered trademark) AK03N (Ajinomoto Healthy Supply Co., Ltd.), mTeSR^{™}1 (STEMCELL Technologies Inc.), and the like. Among them, the culture medium used in the method of the present invention is preferably a medium capable of feeder-free culture and containing a ROCK inhibitor. In particular, when iPS cell is used as the pluripotent stem cell, it is preferable to use a medium such as StemFit (registered trademark) AK03N (Ajinomoto Healthy Supply Co., Ltd.), and the like. Examples of the ROCK inhibitor in the present invention include Y27632, thiazovivin, and the like. In iPS cell culture, Y27632 is generally used as a ROCK inhibitor, and in the present invention, the concentration thereof is preferably 1 to 20 µM, and it is preferable to maintain the added state for 2 days after cell seeding.

The culture period required for sphere formation is not limited as long as spheres are formed. The spheres in the present invention are not of the type formed by forcibly forming spheres by inserting and associating pluripotent stem cells into dimples or the like, but are formed as the cells proliferate, and therefore require a certain period of time. For example, 2 to 10 days from cell seeding are preferred, and 4 to 7 days are more preferred.

The above-mentioned formation of the spheres can be adjusted by the stirring conditions, seeding density conditions, and culture period. The average particle size of the spheres in step (1) of the method of the present invention is generally adjusted to not less than 200 um, specifically within the range of 200 to 600 µm, but is preferably adjusted to 200 to 500 um to further improve the production efficiency of NK cells, and more preferably adjusted to 200 to 400 µm.

When the average particle size is less than 200 um, the number of culture days becomes insufficient and a sufficient number of cells are not obtained, which is undesirable because step (2) is affected. Furthermore, when the average particle size is too large, the supply of sufficient nutrient sources and oxygen to the center of the sphere is blocked, which is undesirable because it induces cell death such as necrosis (e.g., Cells Tissues Organs 196.1 (2012): 34-47).

The sphere formation step (1) is performed by a perfusion culture method. In step (1), the perfusion culture may be performed from the time of seeding the pluripotent stem cells, but it is preferable to perform the perfusion culture about one day after the initial seeding of the pluripotent stem cells, and more preferably, two days after the initial seeding. This is because spheroids are formed after one or two days of culture, and under these conditions the average particle size is not less than 200 um. When the perfusion culture is performed before the formation of spheroids, the spheroids may not be formed satisfactorily.

In addition, in this step (1), the fine pore size of the separation membrane in the perfusion culture is preferably 15 to 75 µm, and more preferably 25 to 45 um. This is because the use of a separation membrane with a pore size not exceeding the desired spheroid size makes it possible to perform the perfusion culture while keeping a certain number of spheroids in the culture container. Therefore, preferably, step (1) includes:
(1-1) a step of forming pluripotent stem cell spheres having an average particle size of 200 µm or more in a first medium; and
(1-2) a step of maintenance culturing the pluripotent stem cell spheres having an average particle size of not less than 200 µm obtained in step (1-1) by a perfusion culture method. That is, the fine pore size of the separation membrane in the perfusion culture in step (1) (or step (1-2)) is preferably 15 to 75 pm, more preferably 25 to 45 µm.

The medium exchange in perfusion culture is performed at a constant dilution rate. The sphere formation process of pluripotent stem cells is preferably performed at a dilution rate of 0.01 to 0.2 hr⁻¹, which can be adjusted with reference to the cell seeding density, the glucose concentration, the lactate concentration, the glutamine concentration and the glutamic acid concentration of the culture supernatant, and the like. The dilution rate can be adjusted by placing the culture container, the supply bottle, and the discharge bottle on a balance or a load cell, or by controlling the rotation speed of the peristaltic pump.

In this step (1), the temperature, pH, and dissolved oxygen concentration can be controlled to any desired value for sphere formation of pluripotent stem cells by using a temperature sensor, a pH sensor, a dissolved oxygen sensor, and the like.

The culture temperature is preferably controlled between 35 and 39°C, more preferably 36 and 38°C. The pH is controlled by the inflow of aseptically treated compressed air, or aseptically treated carbon dioxide gas, or a pH adjuster, or the dilution rate of the perfusion culture medium, and the control value is preferably controlled between 6.8 and 8.0, more preferably 7.0 and 7.4. The dissolved oxygen concentration is controlled by aseptically treated compressed air, or aseptically treated nitrogen gas, or aseptically treated oxygen gas, and the value thereof is controlled between 0 and 6.86 mg/L, and preferably maintained at a concentration of not less than 2.00 mg/L. The gas used to maintain the pH and dissolved oxygen can be blown into the air layer above the culture medium, blown into the culture medium, or exchanged through a gas-permeable membrane, and there is no restriction.

The thus-obtained pluripotent stem cell spheres are subjected to the next step (2) to induce hematopoietic progenitor cells.

### (Step of inducing pluripotent stem cell spheres into cell population including hematopoietic stem cells: step (2))

Step (2) in the method of the present invention is a step of inducing the spheres formed in step (1) into a cell population including hematopoietic progenitor cells by three-dimensional culture using a second medium.

Here, the "cell population including hematopoietic progenitor cells" induced by this step refers to a cell population obtained by step (2), and is a concept that includes a hematopoietic progenitor cell population induced inside or outside the sphere.

This cell population includes hematopoietic progenitor cells formed within the sphere, hematopoietic progenitor cells that have been induced and then escaped from the sphere, and cells inside or outside the sphere that are in the induction process into hematopoietic progenitor cells..

Step (2) is also performed by perfusion culture, as in step (1). The fine pore size of the separation membrane used in this step is preferably 45 to 225 µm, more preferably 45 to 100 um. The perfusion culture in this step is performed from immediately after the start of step (2) until the end of step (2). During this time, the "pluripotent stem cell spheres" are induced toward the "cell population including hematopoietic progenitor cells".

By using a separation membrane with the above-mentioned fine pore size during step (2), perfusion culture can be performed while maintaining a cell population including certain hematopoietic progenitor cells in the culture container.

The medium exchange performed along with the perfusion culture in step (2) is also performed at a certain dilution rate, similar to that described in step (1). Specifically, it is preferably performed at a dilution rate of 0.01 to 0.2 hr⁻¹, similar to step (1), and can be adjusted with reference to the cell seeding density, glucose concentration, lactate concentration, glutamine concentration and glutamic acid concentration of the culture supernatant, and the like. This dilution rate is also applied when the medium used in step (1) is exchanged with the medium used in step (2).

The second medium in step (2) is not particularly limited as long as it is a medium capable of inducing pluripotent stem cells into hematopoietic progenitor cells.

Examples of such medium include a medium containing vascular endothelial growth factor (VEGF), bone morphogenetic protein 4 (BMP4), and a glycogen synthase 3β (GSK3β) inhibitor (hereinafter referred to as medium (2-1)), a medium containing stem cell factor (SCF) and a transforming growth factor β (TGFβ)/Smad inhibitor (hereinafter referred to as medium (2-2)), and a medium containing SCF and Flt3 ligand (Flt3L) (hereinafter referred to as medium (2-3)). It is also possible to design an appropriate induction medium by appropriately changing the combination and amount of each of the medium components as necessary. As examples of such medium, each of the media (2-1) to (2-3) is described below.

The medium (2-1) is a medium containing VEGF, BMP4, and a GSK3β inhibitor.

Here, examples of the GSK3β inhibitor include CHIR99021 and SB216763, and CHIR99021 is preferred.

The concentration of VEGF is preferably 1 to 100 ng/mL, more preferably 50 to 100 ng/mL. The concentration of BMP4 is preferably 1 to 100 ng/mL, more preferably 50 to 100 ng/mL. When CHIR99021 is used, the concentration of the GSK3β inhibitor is preferably 1 to 10 µM, more preferably 1 to 5 µM.

Furthermore, the medium (2-1) may contain a ROCK inhibitor or bFGF. When a ROCK inhibitor is added, examples of the ROCK inhibitor include those described in step (1), and Y27632 is preferred. When Y27632 is used, the concentration of the ROCK inhibitor is preferably 1 to 20 µM, more preferably 1 to 10 µM. When bFGF is added, the concentration is preferably 1 to 100 ng/mL, more preferably 10 to 50 ng/mL.

The basal medium of the medium (2-1) is not particularly limited. For example, media such as DMEM/F-12, HEPES (Thermo Fisher Scientific), and Essential 6 medium (Thermo Fisher Scientific) are preferably used.

The medium (2-2) is a medium containing SCF, a TGFβ/Smad inhibitor, and VEGF.

Here, examples of the TGFβ/Smad inhibitor include SB431542, LY2157299, and LY2109761, and SB431542 is preferred. The concentration of SCF is preferably 1 to 100 ng/mL, more preferably 20 to 50 ng/mL. When SB431542 is used, the concentration of the TGFβ/Smad inhibitor is preferably 1 to 10 µM, more preferably 1 to 5 µM. The concentration of VEGF is preferably 1 to 100 ng/mL, more preferably 50 to 100 ng/mL, similar to the medium (2-1).

Furthermore, the medium (2-2) may contain a ROCK inhibitor or bFGF. Specific examples and concentrations of each when in use are the same as those described for the medium (2-1).

The basal medium for the medium (2-2) is not particularly limited, and the same basal medium as described for the medium (2-1) can be used.

The medium (2-3) is a medium containing SCF and Flt3L. The concentration of SCF is preferably 1 to 100 ng/mL, more preferably 20 to 50 ng/mL. The concentration of Flt3L is preferably 1 to 100 ng/mL, more preferably 20 to 50 ng/mL.

Furthermore, the medium (2-2) may contain at least one selected from a ROCK inhibitor, interleukin (IL)-3, and IL-7. Examples of the ROCK inhibitor include those mentioned above, and Y27632 is preferred. When Y27632 is used, the concentration of the ROCK inhibitor is preferably 1 to 20 µM. When IL-3 is added, the concentration is preferably 1 to 100 ng/mL. When IL-7 is added, the concentration is preferably 1 to 100 ng/mL.

The basal medium for the medium (2-2) is not particularly limited, and a medium suitable for inducing hematopoietic progenitor cells is used. For example, a medium containing Stem Pro-34 SFM (Thermo Fisher Scientific) to which L-glutamine or L-alanyl-L-glutamine has been added to a final concentration of 1 to 10 mM is preferably used.

While the above-mentioned media (2-1), (2-2) and (2-3) can be used alone, it is possible to more efficiently induce hematopoietic progenitor cells by exchanging the medium (2-1) or (2-2) with the medium (2-3) at an appropriate timing during step (2). For example, hematopoietic progenitor cells can be efficiently induced by culturing in the medium (2-1) or (2-2) in the first part of step (2) and in the medium (2-3) in the second part.

In this case, the times for the first part and the second part of step (2) can be appropriately set, and hematopoietic progenitor cells can be efficiently induced by setting, for example, the first part to 2 to 6 days and the second part to 3 to 14 days. The exchange of medium (2-1) or (2-2) with medium (2-3) can be performed by continuous perfusion operation or by replacing the entire amount of the medium with a new medium, and it is more desirable to perform medium exchange by continuous perfusion operation.

Furthermore, hematopoietic progenitor cells can be efficiently induced by exchanging the culture media (2-1) and (2-2) used in the first part of step (2) in this order. Specifically, culture is first performed in medium (2-1), and then in medium (2-2).

In this case, the culture time in each medium can be set appropriately. For example, hematopoietic progenitor cells can be efficiently induced by setting the culture time in medium (2-1) to 1 to 3 days and the culture time in medium (2-2) to 1 to 3 days. This exchange between medium (2-1) and medium (2-2) can be performed by continuous perfusion operation, or by replacing the entire amount of the medium with a new medium, and it is more desirable to perform medium exchange by continuous perfusion operation.

Therefore, the three-dimensional culture in step (2) is preferably a three-dimensional culture including, for example, (2-1) a culture step using medium (2-1) as the second medium, (2-2) a culture step using medium (2-2) as the second medium, and
(2-3) a culture step using medium (2-3) as the second medium. Respective steps (2-1) to (2-3) are preferably performed in this order.

In this step (2), as in step (1), the temperature, pH, and dissolved oxygen concentration can be controlled to any desired value for sphere formation of pluripotent stem cells by using a temperature sensor, a pH sensor, a dissolved oxygen sensor, and the like.

In the method of the present invention, NK cell populations can be obtained without any particular purification step from step (1) to step (3). Therefore, the cell populations obtained in step (2) may be directly subjected to the next step (3) as they are, or only the hematopoietic progenitor cells may be sorted and then subjected to step (3).

The inventors have confirmed that the desired NK cells can be obtained in step (3) by using either method. Therefore, the method of the present invention may include a step of removing cells other than hematopoietic progenitor cells from the "cell population including hematopoietic progenitor cells" obtained in step (2), but it is desirable to subject the "cell population including hematopoietic progenitor cells" obtained in step (2) directly to step (3).

NK cells are induced by subjecting the thus-obtained "cell population including hematopoietic progenitor cells" to step (3).

### (Step of inducing hematopoietic progenitor cell into NK cell: step (3))

Step (3) in the method of the present invention is a step of inducing the "cell population including hematopoietic progenitor cells" obtained in step (2) into a "cell population including NK cells" by three-dimensional culture using a third medium.

Here, the "cell population including NK cells" induced by this step refers to a cell population containing NK cells obtained by step (3) and cells in the induction process into NK cells.

NK cells generally exist as single cells without forming spheres. Therefore, by step (3), the single NK cells proliferates to become a cell population of many single NK cells. This cell population contains the majority of NK cells and some cells in the induction process into NK cells.

Step (3) can also be performed by perfusion culture, as in steps (1) and (2). The fine pore size of the separation membrane to be used is preferably 0.1 to 10 µm, more preferably 0.2 to 5 um. The perfusion culture in this step is performed continuously from immediately after the start of step (3) until the end of step (3). During this time, NK cells induced from the hematopoietic progenitor cell spheres are generated, and the NK cells do not remain within the spheres but are individually filtered out. Therefore, by using a separation membrane that does not exceed the cell size of the NK cells during step (3), perfusion culture can be performed while keeping a certain number of NK cells in the culture container.

Furthermore, the medium exchange in this step (3) is also performed at a constant dilution rate as described in step (1). Specifically, it is preferably performed at a dilution rate of 0.01 to 0.2 hr⁻¹, similar to step (1), and can be adjusted with reference to the cell seeding density, glucose concentration, lactate concentration, glutamine concentration and glutamic acid concentration of the culture supernatant, and the like. This dilution rate is also applied when the medium used in step (2) is exchanged with the medium used in step (3).

The third medium in step (3) is not particularly limited as long as it is a medium capable of inducing hematopoietic progenitor cells into NK cells. Examples of such a medium include a medium containing IL-15 and SCF. The concentration of IL-15 is preferably 1 to 100 ng/mL, more preferably 20 to 50 ng/mL. The concentration of SCF is preferably 1 to 100 ng/mL, more preferably 20 to 50 ng/mL. This medium may further contain one or more components selected from IL-7, Flt3L, a ROCK inhibitor, a GSK3β inhibitor, and a TGFβ receptor (TGFβR) inhibitor. Examples of the ROCK inhibitor include Y27632 and thiazovivin, and Y27632 is preferred. Examples of the GSK3β inhibitor include CHIR99021 and SB216763, and CHIR99021 is preferred. Examples of the TGFβR inhibitor include LY2157299, SB431542, and LY2109761, and LY2157299 is preferred. In a preferred embodiment, the third medium further contains IL-7 and Flt3L in addition to IL-15 and SCF.

When IL-7 is added, the concentration is preferably 1 to 100 ng/mL, more preferably 20 to 50 ng/mL. When Flt3L is added, the concentration is preferably 1 to 100 ng/mL, more preferably 20 to 50 ng/mL. When a ROCK inhibitor is added, the concentration is preferably 1 to 20 µM, more preferably 1 to 10 µM. When a GSK3β inhibitor is added, the concentration is preferably 1 to 10 nM, more preferably 1 to 5 µM. When a TGFβR inhibitor is added, the concentration is preferably 0.1 to 100 µM, more preferably 0.1 to 5 µM.

The culture period in step (3) is generally 20 to 60 days, preferably about 25 to 40 days.

In addition, when IL-7, Flt3L, a ROCK inhibitor, a GSK3β inhibitor, or a TGFβR inhibitor is added in step (3), the induction efficiency can be improved by adjusting the timing of adding these factors. For example, IL-7 and Flt3L are preferably removed on or after the 10th day of culture in step (3), more preferably on or after the 20th day. In addition, a ROCK inhibitor, a GSK3β inhibitor, and a TGFβR inhibitor are preferably added for 4 to 7 days before the end of culture.

The basal medium to be used in step (3) is not particularly limited. For example, media such as AIM-V Medium (Thermo Fisher Scientific), Stemline (registered trademark) II (Sigma-Aldrich), ALyS505N-0 (Cell Science Institute, Inc.), and Stem Pro-34 SFM (Thermo Fisher Scientific) are used, and AIM-V Medium is preferably used. Furthermore, the medium may contain human serum, fetal bovine serum (FBS), or a serum replacement. The concentration thereof is preferably 1 to 20%, more preferably 1 to 10%.

In step (3), it is preferable to further perform a step of removing the spheres. The timing of removal may be, for example, before the start of this step, during this step, or at the end of this step, but it is not particularly limited and can be performed at any time.

The spheres are preferably removed using a 20 to 100 µm cell strainer, and more preferably removed using a 20 to 40 µm cell strainer.

In this step (3), as in step (1), the temperature, pH, and dissolved oxygen concentration can be controlled to any desired value for sphere formation of pluripotent stem cells by using a temperature sensor, a pH sensor, a dissolved oxygen sensor, and the like.

In the method of the present invention, a step of expansion culture of the obtained NK cells or a step of maturation of NK cells may be further performed as step (4) after step (3). The expansion culture step or the maturation step can be performed by appropriately applying a known method.

The method of the present invention is characterized in that all of the above-mentioned steps (1) to (3) or (1) to (4) are performed without using a carrier for three-dimensional culture or an extracellular substrate.

The method of the present invention may further include a step of cryopreserving the obtained NK cells. After completion of the NK production step, the NK cells of the present invention can be frozen and preserved by a method known per se.

The present invention is described in more detail in the following with reference to Example and Experimental Example. However, they are merely illustrative and the present invention is not limited thereto..

### [Example]

### Example 1: Induction of differentiation from pluripotent stem cells into NK cells by perfusion culture

In this example, human iPS cell line 06E (TC-1133HKK_06E_MCB) was used as the pluripotent stem cell, and the process from iPS cell sphere formation to NK cell differentiation was performed by a three-dimensional perfusion culture method. A stirring blade was used to mix the culture medium, and a pH sensor, a temperature sensor, and a dissolved oxygen sensor were used as sensors, and various management items were continuously monitored and controlled while mixing the culture medium. In addition, as shown in Fig. 1, the perfusion culture was managed by controlling the inflow and outflow rate per hour using a dedicated pump and a balance.

### step (1): Step of forming pluripotent stem cell sphere

For formation of pluripotent stem cell spheres, iPS cells expanded in two dimensions were seeded into a three-dimensional culture container at a cell density of 1.0×10⁵ cells/mL. Other details are as in the following method:
The medium used in step (1) was StemFit (registered trademark) AK03N (Ajinomoto Healthy Supply Co., Ltd.) supplemented with the ROCK inhibitor Y27632 at 10 µM.

The culture container was a single-use bottle with a total volume of 500 mL, and the culture medium was continuously mixed using a stirring blade inside the container. The pH was continuously monitored with a pH sensor and controlled to 7.07 by supplying sterile medium, sterile air, or sterile carbon dioxide.

The dissolved oxygen concentration was continuously monitored using a dissolved oxygen sensor and controlled to maintain a concentration of not less than 1.50 mg/L by supplying sterile air or sterile oxygen gas. The culture temperature was continuously monitored using a temperature sensor and controlled to 37.0°C by heating the container from the outside with a heater. The culture medium volume was adjusted to 250 mL, and a fresh medium was supplied using a pump and a balance, and the culture medium was discharged at the same rate. The dilution rate was controlled at 0.02 hr⁻¹ from the second day to the fourth day of culture, and 0.03 hr⁻¹ from the fourth day to the seventh day of culture. The separation membrane used was made of SUS316 with a fine pore size of 45 µm.

As comparison targets, two types of samples in which the medium was changed manually were prepared. Two samples, one with a culture medium volume of 250 mL and the other with a culture medium volume of 30 mL, were prepared for comparison, and the medium exchange was performed manually.

In this case, medium exchange was performed by stopping the stirring of the culture container, standing same for 10 min, and then changing 50% of the supernatant on day 2, and 80% on days 4, 5, and 6. Samples with a 30 mL culture medium volume were cultured in a rotary stirring container, and floating culture was performed in a CO₂ incubator at 5% CO₂ concentration and at 37°C. The culture medium was mixed and cultured at a rotation speed of 55 rpm. Samples with a 250 mL culture medium volume were cultured under the same control conditions as the perfusion culture samples, except that the medium exchange was performed manually.

On the seventh day of culture, the cell suspension containing spheres was collected from the culture medium, and the average particle size of the spheres was measured using a microscope. Microscopic photographs of the spheres (4x objective lens) and the average particle size of the spheres are shown in Fig. 2. The 250 mL culture allowed the spheres to be created with a controlled diameter between 200 and 400 um. In addition, when perfusion culture was performed, the medium exchange that occurs during the process was continuously and automatically controlled, and uniform spheres were formed without any problems, without moving the culture tank to a sterile environment such as a safety cabinet and the like.

### step (2): Step of inducing pluripotent stem cell spheres into cell population including hematopoietic progenitor cells

To produce hematopoietic progenitor cells, iPS cell spheres produced in step (1) were seeded in a three-dimensional culture container to a cell density of 1.8×10⁵ cells/mL. Other details were as in the following method.

The medium used was DMEM/F-12, HEPES (Thermo Fisher Scientific) supplemented with CHIR99021 at 2 µM, BMP4 at 80 ng/mL, VEGF165 at 80 ng/mL, and bFGF at 50 ng/mL until day 2, and Essential 6 (Thermo Fisher Scientific) supplemented with SCF at 50 ng/mL, VEGF165 at 80 ng/mL, SB431542 at 2 µM, and bFGF at 50 ng/mL from day 2 to day 4. Furthermore, from day 4 to day 14, a general hematopoietic progenitor cell differentiation-inducing basal medium supplemented with SCF at 50 ng/mL and Flt3L at 50 ng/mL was used.

The culture container was a single-use bottle with a total volume of 500 mL, and the culture medium was continuously mixed using a stirring blade inside the container. The pH was continuously monitored with a pH sensor and controlled to 7.07 by supplying sterile medium, sterile air, or sterile carbon dioxide.

The dissolved oxygen concentration was continuously monitored using a dissolved oxygen sensor and controlled to maintain a concentration of not less than 1.50 mg/L by supplying sterile air or sterile oxygen gas. The culture temperature was continuously monitored using a temperature sensor and controlled to 37.0°C by heating the container from the outside with a heater. The culture medium volume was adjusted to 250 mL, and a fresh medium was supplied using a pump and a balance, and the culture medium was discharged at the same rate. The dilution rate was controlled at 0.07 hr⁻¹ from the first day to the second day of culture, 0.06 hr⁻¹ from the second day to the fourth day of culture, 0.13 hr⁻¹ from the fifth day to the seventh day of culture, 0.07 hr⁻¹ from the seventh day to the twelfth day of culture, and 0.02 hr⁻¹ from the twelfth day to the fourteenth day of culture. The separation membrane used was made of SUS316 with a fine pore size of 75 µm.

As comparison targets, two types of samples in which the medium was changed manually were prepared. Two samples, one with a culture medium volume of 250 mL and the other with a culture medium volume of 30 mL, were prepared for comparison, and the medium exchange was performed manually. As a method for medium exchange, the medium exchange was performed by stopping the stirring of the culture container, standing same for several min, and changing the total amount of the supernatant on days 1, 2, 3, 4, 7, 9, and 12. Samples with a 30 mL culture medium volume were cultured in a rotary stirring container, cultured in a CO₂ incubator, and floating cultured at 5%, 37°C. The culture medium was mixed and cultured at a rotation speed of 55 rpm. Samples with a 250 mL culture medium volume were cultured under similar control as the perfusion culture samples, except that the medium exchange was performed manually.

On the 14th day of culture, the spheres were removed from the culture medium to obtain a suspension of floating cells, and the cell surface markers known to be expressed in hematopoietic progenitor cells (CD34, CD43, CD45, CD117) were confirmed by flow cytometry (Fig. 3).

As a result, the production efficiency of hematopoietic progenitor cells was compared between the production by perfusion culture and the manual medium exchange. As a result, in this step, the medium exchange that occurs during the process was continuously performed under automatic control, the culture could be performed without moving the culture tank to a sterile environment such as a safety cabinet, and there was no significant difference in the expression of the confirmed markers. Therefore, hematopoietic progenitor cells could be induced by perfusion culture in step (2).

### step (3): Step of inducing hematopoietic stem cell into natural killer cell

NK cells were produced by seeding the hematopoietic progenitor cells produced in step (2) (floating cells containing spheres) into a three-dimensional culture container at a cell density of 1.0×10⁵ cells/mL. In addition, subculture was performed when the cell density reached not less than 1.0×10⁷ cells/mL as a result of the culture. After subculture, the culture was continued in the same medium and culture conditions until the cell density reached not less than 2.0×10⁷ cells/mL. Other details were as in the following method.

The medium used was AIM V Serum Free Medium (Thermo Fisher Scientific) with FBS added at a concentration of 5%, to which IL-15, IL-7, SCF, and Flt3L were added to a final concentration of 50 ng/mL.

The culture container was a single-use bottle with a total volume of 500 mL, and the culture medium was continuously mixed using a stirring blade inside the container. The pH was continuously monitored with a pH sensor and controlled to 7.07 by supplying sterile medium, sterile air, or sterile carbon dioxide.

The dissolved oxygen concentration was continuously monitored using a dissolved oxygen sensor and controlled to maintain a concentration of not less than 1.50 mg/L by supplying sterile air or sterile oxygen gas. The culture temperature was continuously monitored using a temperature sensor and controlled to 37.0°C by heating the container from the outside with a heater. The culture medium volume was adjusted to 250 mL, and a fresh medium was supplied using a pump and a balance, and the culture medium was discharged at the same rate. The dilution rate was controlled from 0.01 hr⁻¹ to 0.09 hr⁻¹ according to the cell density.

A polysulfone fiber product with a pore size of 7 um was used for the separation membrane. As comparison targets, manual medium exchange was performed on two samples, one with a culture medium volume of 250 mL and the other with a culture medium volume of 30 mL. According to manual medium exchange, the medium was exchanged once every 1-2 days. Specifically, after stopping the stirring of the culture container, 50-90% of the culture medium was recovered according to the cell density, and centrifuged under the conditions of 20°C, 300 g, 5 min. The supernatant was removed, and a new medium in an amount equal to the amount of the removed medium was added to the remaining pellet, mixed well, and then returned to the culture container.

For the sample with a culture medium volume of 30 mL, suspension culture was performed using a rotary stirring container at 55 rpm in a 5% CO₂ incubator at 37°C. Samples with a 250 mL culture medium volume were cultured under the same control as the perfusion culture samples, except that the medium exchange was performed manually.

100 µL of the culture medium was collected during the culture, and the density of the floating cells was measured using NC-200 (ChemoMetec) (Fig. 4). By adopting the method of the present invention, it was possible to.culture the cells at a higher cell density after subculture than the comparison target. Since the medium exchange during this culture step was performed continuously and automatically, it was shown that cells can be cultured at a higher density while reducing the workload.

The NK cell culture solution produced by the method of the present invention was collected and centrifuged at 20°C, 300 g for 5 min. The supernatant was removed, and the cells were resuspended in STEM-CELLBANKER (registered trademark) GMP grade, transferred to a -80°C freezer, and frozen. The frozen cells were removed from the freezer and thawed in a 37°C water bath. They were then resuspended in a medium containing AIM V Serum Free Medium (Thermo Fisher Scientific) with FBS added at a concentration of 5%, and IL-15, IL-7, SCF, and Flt3L at a final concentration of 50 ng/mL. The cells were centrifuged at 20°C, 300 g, and 5 min. After removing the supernatant, the cells were resuspended again in the same medium, seeded in a T75 flask, and static culture was performed in a CO₂ incubator controlled at 5% and 37°C. The cells were harvested after 3 days, and the expression of CD56 as a cell surface marker known to be expressed in NK cells, and granzyme B and perforin known to be involved in the cytotoxic activity of NK cells was confirmed by flow cytometry (Fig. 5). As a result, the expression of CD56, granzyme B, and perforin could be confirmed as in the comparison target, demonstrating that the method of the present invention can produce natural killer cells with properties that are the same as those by existing methods.

### Experimental Example 1: Activity evaluation of obtained NK cell

The cells obtained in Example 1 and after freezing, thawing, and static culture were co-cultured with A549 cells, which are human alveolar basal epithelial adenocarcinoma cells (ET ratio 3:1). Lactate dehydrogenase (LDH) activity in the culture solution was measured 4 hr after co-culture to determine the cytotoxic activity of the produced NK cells against A549 cells. As a comparison target, NK cells sorted from human peripheral blood mononuclear cells and cultured (primary NK) were also treated in the same way (Fig. 6).

As a result, the NK cells produced from iPS cells showed higher cytotoxic activity than the primary NK cells, and the NK cells produced using perfusion culture which is the method of the present invention showed cytotoxic activity equivalent to that by existing culture methods.

### [Industrial Applicability]

The method for producing NK cells derived from pluripotent stem cell of the present invention performs medium exchange during each step continuously under automatic control, can perform mass production without moving the culture tank to a sterile environment such as a safety cabinet, can perform spheroid formation and cell differentiation in each step without problems as compared with existing methods, and has been shown to enable higher density culture in high-density culture of NK cells. Therefore, it can be said that this method is useful for mass production of NK cells that can be used in cancer immunotherapy.

This application is based on a patent application No. 2022-013646 filed in Japan (filing date: January 31, 2022), the contents of which are incorporated in full herein.

## Claims

1. A method for producing a natural killer (NK) cell, comprising
(1) a step of forming pluripotent stem cell spheres having an average particle size of not less than 200 µm in a first medium;
(2) a step of inducing the pluripotent stem cell spheres formed in step (1) into a cell population including hematopoietic progenitor cells by three-dimensional culture using a second medium; and
(3) a step of inducing the cell population including hematopoietic progenitor cells obtained in step (2) into a cell population including NK cells by three-dimensional culture using a third medium,
wherein the steps (1) to (3) are performed by a perfusion culture method.

2. The method according to claim 1, wherein the steps (1) to (3) are performed without using a three-dimensional culture carrier or an extracellular substrate.

3. The method according to claim 2, wherein the perfusion culture in step (1) is performed using a separation membrane having a fine pore size of 15 - 75 µm.

4. The method according to claim 2, wherein the perfusion culture in step (2) is performed using a separation membrane having a fine pore size of 45 - 225 µm.

5. The method according to claim 2, wherein the perfusion culture in step (3) is performed using a separation membrane having a fine pore size of 0.2 - 10 µm.

6. The method according to any one of claims 1 to 5, wherein the steps (1) to (3) are performed by a continuous perfusion culture method.

7. The method according to claim 1, wherein the first medium comprises a ROCK inhibitor.

8. The method according to claim 1, wherein the second medium comprises VEGF, BMP4, and a GSK3β inhibitor.

9. The method according to claim 8, wherein the second medium further comprises a ROCK inhibitor or bFGF.

10. The method according to claim 1, wherein the second medium comprises SCF, a TGFβ/Smad inhibitor, and VEGF.

11. The method according to claim 10, wherein the second medium further comprises a ROCK inhibitor or bFGF.

12. The method according to claim 1, wherein the second medium comprises SCF and Flt3L.

13. The method according to claim 12, wherein the second medium further comprises at least one selected from a ROCK inhibitor, IL-3, and IL-7.

14. The method according to claim 1, wherein the three-dimensional culture in step (2) comprises
(2-1) a culture step using a medium comprising VEGF, BMP4, and a GSK3β inhibitor as the second medium,
(2-2) a culture step using a medium comprising SCF, a TGFβ/Smad inhibitor, and VEGF as the second medium, and
(2-3) a culture step using a medium comprising SCF and Flt3L as the second medium.

15. The method according to claim 1, wherein the third medium comprises IL-15 and SCF.

16. The method according to claim 15, wherein the third medium further comprises IL-7 and Flt3L.

17. The method according to claim 1, which does not require a step of sorting NK cells.
